# EUROPEAN PATENT APPLICATION

(11) **EP 2 315 023 A1**
(43) Date of publication of application: **27.04.2011**
(21) Application number: 09450201.0
(22) Date of filing: 21.10.2009
(51) Int. Cl.: G01N 33/34

(54) **Process for quantitative determination of emissions, consisting of particulate matter, from surfaces of flexible sheet-like materials and device therefore**

(71) Applicant: Mondi Uncoated Fine & Kraft Paper GmbH, 1032 Wien (AT)
(72) Inventor: Kornherr, Andreas, 1100 Vienna (AT); Steiner, Gerhard, 1170 Vienna (AT); Reischl, Georg, Perchtoldsdorf (AT)
(74) Representative: Cunow, Gerda

(57) **Abstract**

The present invention is directed to a process for quantitative determination of emissions, consisting of particulate matter, from surfaces of flexible sheet-like materials, wherein the flexible sheet-like material is fixed in a frame (4), introduced in a measurement chamber (18), flushed with highly-purified air and periodically moved by exposing to a mechanical stress, whereupon the particulate matter emitted by the flexible sheet-material is fed by highly-purified air to a detection device, whereby the mechanical stress is generated by waves, especially pressure waves or sound waves. Furthermore a device for quantitative determination of emissions consisting of particulate matter is also provided. (Fig. 2)

## Description

The present invention is directed to a process for the quantitative determination of emissions, consisting of particulate matter, from surfaces of flexible sheet-like materials, wherein the flexible sheet-like material is fixed in a frame, introduced in a measurement chamber, flushed with highly-purified air and periodically moved by exposing to a mechanical stress, whereupon the particulate matter emitted by the flexible sheet-like material is fed by highly-purified air to a detection device. Furthermore the present invention is directed to a device for quantitative determination of emissions, consisting of particulate matter, from surfaces of a flexible sheet-like material, comprising a casing, in which casing a frame for holding the flexible sheet-like material is introducible, at least one supply conduit for highly-purified air and at least one discharge conduit for a stream, consisting of highly-purified air and emitted particulate material, a mechanical stress generating device and a detection and measurement unit.

In known processes for a quantitative or also qualitative determination of particle emissions from surfaces of flexible sheet-like materials, such as paper, plastics or also fabrics the flexible sheet-like material, which is hold in a frame is introduced in a sealed measurement chamber and moved by a stamp of a linear induction motor for emitting the particulate matter. The emitted particulate matter is fed together with a carrier gas, for example purified air, to a detection device for measuring the quantity and the quality of the particles emitted. Such a device is for example described in "papierausösterreich" No. 6/08, page 32 ff. of the applicant of the present application and wherein a device for measuring particles, which are adhered on paper or contained in paper or card board, is investigated.

In this known device the signal to noise ratio is low and moreover the measurement time for obtaining any result is long. In addition the stamp of the linear induction motor used can lead to emissions of particles in the measurement chamber, which disturbs and falsifies the measurement.

It is therefore an object of the present invention to develop a process for a quantitative determination of particle emissions, in which process the drawbacks of a known process are avoided and which process can especially provide excellent results in short time.

For achieving this object the process of the present invention is characterized in that the mechanical stress is generated by waves, especially pressure waves or sound waves. By generating the mechanical stress by waves, especially pressure waves or sound waves it is possible to reduce the time necessary for the measurement of particles in a great extent, because of the fact that the frequency of the pressure waves or sound waves can be chosen according to the requirements of the flexible sheet-like materials to be measured. A further enhancement of the mechanical stress generated by waves can be obtained with waves showing a specific wave form. With such a specific wave form a further enhancement of the defined mechanical stress is obtainable and therefore the particles adhered on or contained in the flexible sheet-like material are emitted faster and to a higher extent, which makes the whole process exacter.

A further saving of time can achieved with a process, which is characterized in that the mechanical stress is exhibited on both sides of the frame by at least one wave-generating device on each side. By exhibiting the mechanical stress on both sides of the frame it is made sure that nearly all particles, which can be emitted from the flexible sheet-like material, are nearly immediately separated from the surface of the flexible sheet-like material and it is therefore not only possible to obtain any result in a short time but also to obtain an exact and quantitative result showing the whole content of separable particles, especially nanoparticles, which are contained on or in a tested flexible sheet-like material.

A further enhancement of the process according to the present invention can be achieved if the waves of the wave-generating devices, which are arranged on opposite sides of the frame, are shifted by phase, especially shifted by 180°. If the two signals generated by the wave generating devices are shifted by phase, especially shifted by 180° (π) the transmitted acoustic or pressure waves will sum up at the position of the sample and enhance therefore the efficiency of the system, in which case the efficiency of the process is improved and the time necessary further shortened.

According to a preferred embodiment of the present invention the frequency and/or the amplitude of the waves are continuously controlled. By continuously controlling the frequency and/or the amplitude of the waves it is possible to investigate the emission characteristics of the particles over a broad spectrum of intensity and the movement pattern of the mechanical stress applied to the flexible sheet-like material.

A further improvement of the process according to the present invention can be obtained, if energy generated by the wave generating device(s) releases attached or absorbed particles, with a particle size of at least 2 nm, from the surface of the flexible sheet-like material. Especially small particles, namely particles with particle sizes of at least 2 nm are very strongly attached or absorbed on the surface of flexible sheet-like materials and it was surprisingly found that the energy generated by the wave generating devices according to the present invention releases efficiently and nearly completely such absorbed or attached nanoparticles.

According to a preferred embodiment of the present invention the process is characterized in that a continuous stream of highly-purified air is maintained during the exposure to mechanical stress and that after ending of the exposure to mechanical stress, the measurement chamber is further flushed with highly-purified air. If a continuous stream of highly-purified air is maintained during the exposure to mechanical stress and after the ending of the exposure to mechanical stress it is made sure that all particles emitted from the surface of the material to be tested are fed to the detection device and therefore not only a correct result can be obtained but also the noise reduction can be.

A further improvement of the process according to the present invention can be obtained, if a stream of highly-purified air, which is enriched with positive and negative ions, for transporting the particulate matter emitted by the flexible sheet-like material, is continuously introduced into the measurement chamber. If the flushing air is enriched with positive or negative ions it is possible to neutralize the surface charges on the sample and on all surfaces of the measurement chamber and thereby measurement artifacts caused by electrostatic effects can be avoided. The best results can be obtained with a concentration of positive or negative ions in the stream of highly-purified air of at least 10⁵ ions/cm³, especially 10⁷ ions/cm³. With such a relatively high concentration of positive and negative ions the above-mentioned effects are minimal, especially measurement artifacts are totally avoided and therefore the signal to noise ratio is further improved.

The best results with the process according to the present invention can be obtained, if the flexible sheet-like material is selected from the group consisting of paper, cardboard, synthetic materials, leather, foils, cloth or fabric. By selecting the flexible sheet-like material from the group consisting of paper, cardboard, synthetic materials, leather, cloth or fabric it is possible to conduct the process according to the present invention such that particulate matter with a particle size of at least 2 nm can be fed to the detection device and detected therein. With all already known processes it is not possible to detect particle-emissions with particle sizes being so small and moreover it is not possible to obtain results, in which the signal to noise ratio is as high as in the process according to the present invention.

It is a further object of the present invention to provide a device for quantitative determination of emissions, consisting of particulate matter, from surfaces of a flexible sheet-like material, comprising a casing, in which casing a frame for holding the flexible sheet-like material is introducible, at least one supply conduit for highly-purified air and at least one discharge conduit for a stream, consisting of highly-purified air and emitted particulate material, a mechanical stress generating device and a detection and measurement unit for obtaining precise results in a short time.

For achieving this object of the present invention the device for quantitative and qualitative determination of emissions consisting of particulate matter from surfaces of a flexible sheet-like material is characterized in that the mechanical stress generating device is selected from a pneumatic system or a sound waves producing system. A device, in which the mechanical stress generating device is selected from a pneumatic system and a sound waves producing system can be constructed in a very simple manner and especially only totally smooth surfaces can be arranged in a measuring chamber so that especially the emission of particulate matter, which adheres on the materials or is contained in material building up the measurement chamber can be avoided or at least minimized.

According to an embodiment of the present invention the device is characterized in that the casing comprises at least two parts, which are air tight connectable and that in at least one part one element of at least one mechanical stress generating device is contained. With a casing, which comprises a least two parts which are air tight connectable it is possible to construct a device with only flat surfaces and furthermore the only seal or gasket necessary for an air tight closure of the device has a simple form and can also be made of materials, which do not emit nanoparticles or microparticles and therefore the signal to noise ratio can be further improved.

Because of the fact that in least one part an element with at least one mechanical stress generating device is contained it this furthermore possible to build a device generating mechanical stress in only one part of the device and for example to construct the mechanical stress generating device in such a manner that only a flat surface, for example a membrane made of synthetic material is directed to the flexible sheet-like material to be measured. If the membrane made of synthetic material is made of a material, which does not emit particles, especially does not emit nano- or macroparticles it is possible to have a very simple construction of the measurement chamber and at the same time it is possible to further reduce the emission of particles, which are not contained in the flexible sheet-like material to be tested, i.e. of artifacts.

For obtaining precise measurement results in a short time a device according to the present invention contains as mechanical stress generating device at least two speakers. If the device contains at least two speakers as mechanical stress generating devices it is especially possible to provide a measurement chamber, which has except the material to be tested, only flat surfaces consisting of particle emission free membranes made of synthetic materials, wherein also the speaker membranes made of such material. With such a device the signal to noise ratio is excellent and therefore the measuring accuracy is improved.

By providing a device, which is characterized in that in one part of the casing a frame holding device is provided, in which frame holding device the frame is introducible or removable by a frame exchange mechanism, it is possible to perform a series of measurements in short time only by removing the frame with material already measured and introducing a new one, in which the flexible sheet-like material to be measured is contained.

According to an embodiment of the present invention the measurement unit contains different aerosol characterizing and sampling instruments, which are selected from a condensation particle counter, an electro-mobility spectrometer, a flow control unit, an electrostatic precipitator, an electron microscope and particle sampling elements, such as particle filters or so on. With such different measurement units not only a quantitative and qualitative determination of the particulate matter emissions is possible, but also for example also a chemical analysis of the particles emitted. It is therefore also possible to estimate whether or not the particles emitted are harmful or not.

In the following the invention is further described on the basis of a drawing wherein:
Fig. 1 shows a block diagram of a device for quantitative determination of emissions consisting of particulate matter from surfaces of a flexible sheet-like material,
Fig. 2 shows a diagrammatic view of the measurement unit containing a speaker as mechanical stress generating device;
Fig. 3 shows an axial section of an analogous device containing two speakers, and
Fig. 4 shows a graph of the particle size distribution, obtained with a device according to the present invention.

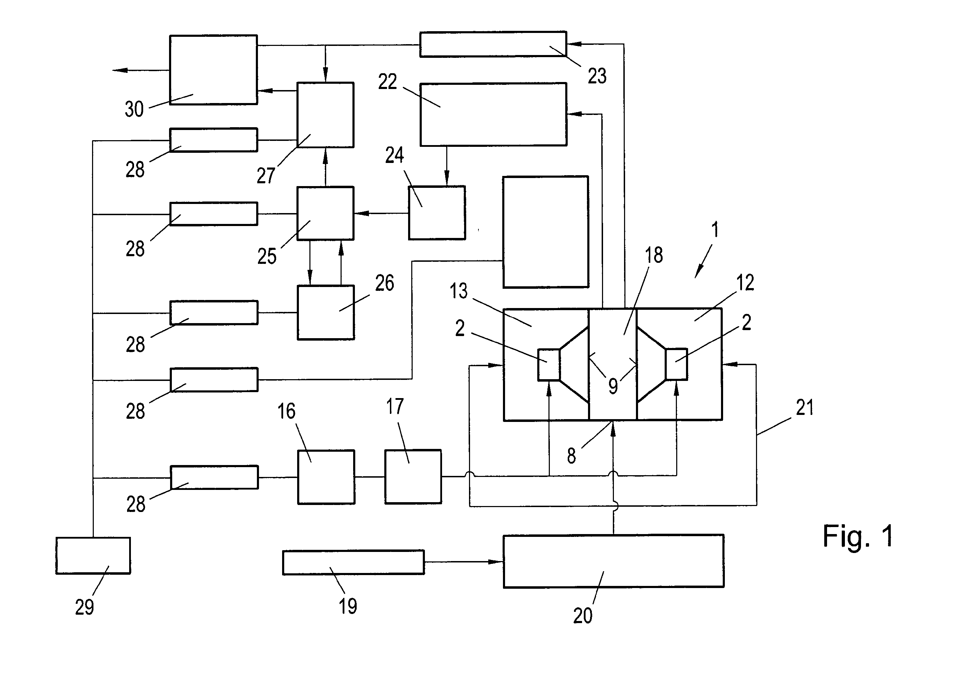

In Fig. 1 a block diagram for a device according to the present invention for quantitative and qualitative determination particulate emissions is shown, wherein a casing containing a measurement chamber, two speakers and the material to be tested is designated with 1. This casing 1 is divided in three parts, wherein parts 12 and 13 are identically and in the middle between parts 12 and 13 a measurement chamber 18 is formed. In the parts 12 and 13 two speakers 2 are contained, wherein the speaker membrane 9 of each speaker 2 is directed to the measurement chamber 18. The measurement chamber 18 is flushed with highly-purified air, which air is delivered into the measurement chamber 18 from a source of oil-free pressurized air 19, which air is directed through a conditioner 20 through holes 8, which are not shown in Fig. 1.

In the interior of the measurement chamber 18 a sample to be tested is contained, which is not shown in Fig. 1. For measuring the amount of particulate matter contained in the material to be tested, this material is admitted with sound waves from the speakers 2, wherein the sound waves are shifted by phase, which is schematically shown by an arrow 21. After admitting the material to be tested with sound waves highly-purified air together with particles, especially nanoparticles, emitted by the material to be tested are fed to an aerosol sampling device 22 and to a plenum chamber 23, wherefrom air enriched with particulate matter is fed in the case of the aerosol sampling device 22 for example to a neutralizing device 24. Thereafter the stream to be analysed is fed to a differential mobility analyzer 25, for example, and optionally to a flow control unit 26.

The gas, which was fed to the plenum chamber 23 is in the following fed to a condensation particle counter 27, which particle counter can also be fed with a stream from the differential mobility analyzer 25. After having performed all measurements in the units 25, 26 and 27 the measurement results of these units are directed through interfaces 28 to a controlling Personal Computer 29.

Exhaust gas will be fed to an exhaust filter system 30 for filtering off particulate matter, especially nanoparticles, and the purified air is then discharged into the environment.

For controlling the function of the speakers 2 and especially for controlling the frequency and the amplitude of the speakers 2 the speakers 2 are also controlled by the controlling Personal Computer 29. Also in this case the controlling PC controls a function of speakers 2 through an interface 28 and for example a remote controlled function generator 16 and a power amplifier 17, which can be of any known type and also for example an two channel subwoofer power amplifier.

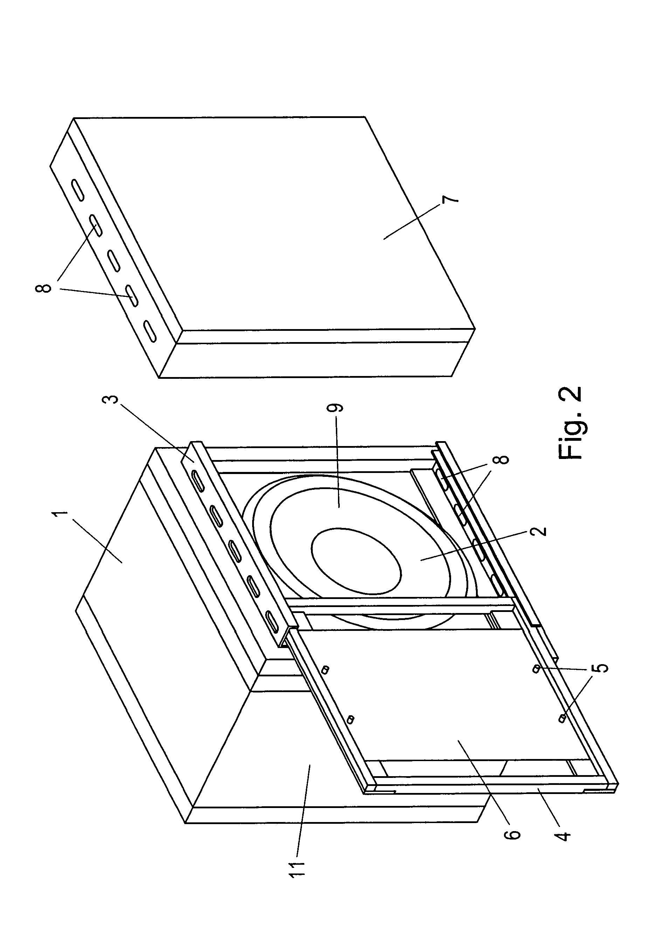

In Fig. 2 a casing 1 containing a speaker 2 is equipped with a holding device 3 on its front part. In the holding device 3 a frame 4 containing the flexible sheet-like material to be tested can be introduced and removed by a simple exchange mechanism. In the frame 4 a holding mechanism, which is schematically indicated with 5 is provided. The flexible sheet-like material to be tested is fixed on the holding mechanism 5 in such a manner that the free moving part 6 of the flexible sheet-like material to be tested can move about 3 cm from the middle position without touching the holding mechanism, the speaker 2 or the casing 1. The casing 1 is furthermore provided with a cover 7, which cover 7 contains openings or holes or parts 8 for feeding and discharging the highly-purified air on its basis and on its top surface, forming the carrier gas for the emitted particles to be tested, especially for the emitted nanoparticles.

In the casing 1 a speaker 2 is built in and is equipped with a speaker membrane 9 made of synthetic material, which does not show any particle emission. On the back side of the casing 1 an exit 10 is provided, which is not visible in Fig. 2, for being able to merge the acoustic waves exiting at the back of the speaker compartment 11, wherein this exit 10 is provided with an air tight tubing to insulate the whole system from the ambient laboratory air.

For measuring the particle emission of the flexible sheet-like material the frame 4 is introduced into the holding mechanism 3 in such a manner that the flexible sheet-like material is localized in the very centre before the speaker membrane 9, whereafter the cover 7 is put on the casing 1 and the whole system will be closed in air tightly manner.

After having closed the casing 1 the casing 1 will be flushed with preconditioned purified clean air as long as particles can be detected in the measurement chamber 18. The purified air is introduced through the holes 8, which are provided in the bottom of the casing 1 in the holding mechanism 3 as well as in the cover 7, wherein the holes 8 are provided in such way that the holes 8 does exactly flush.

In an analogous manner holes 8 for discharging the air are provided in the top region of the holding mechanism 3 and of the casing 1, which is enriched with particles. For avoiding the measurement of artifacts caused by electrostatic effects the flushing air is enriched with approximately 10⁷/cm³ of positive and negative ions to neutralize surface charges on the sample and on the interior faces of the measurement chamber.

After having flushed the measurement chamber with highly-purified air a periodic electric signal is applied to the speaker and the air column of the speaker membrane causes vibrations of the sample and deforms it mechanically. The acoustic waves exiting at the back side of the casing 1 especially through the tubing 10, which tubing 10 is provided in such a manner that it insulates the whole system from the ambient laboratory air. Such an insulation is of great importance as the estimated particle concentrations emitted by the sample surfaces under investigation are up to 3 to 5 orders of magnitude lower compared to typical indoor particle concentrations.

In the following the exit air from the measurement chamber 8 will be guided to various aerosol characterizing and sampling instruments, which are not shown in the present figure.

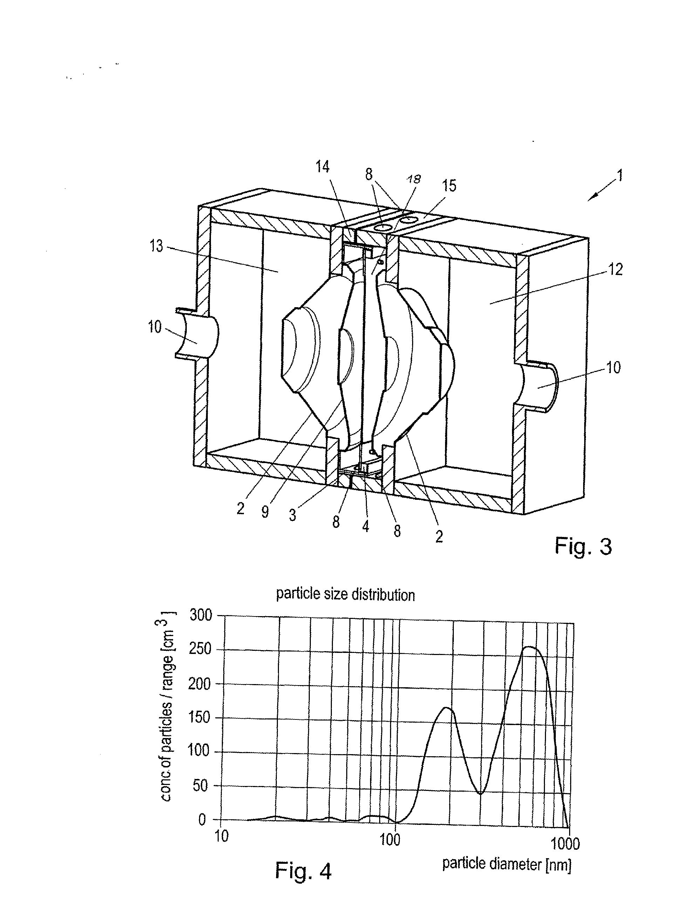

In Fig. 3 an analogous device is shown, wherein in Fig. 3 the casing 1 has two parts 12 and 13, which parts 12 and 13 are each equipped with a speaker 2 and in the middle between the two parts 12 and 13 of the casing 1 an intermediate part 14 is provided, which intermediate part 14 forms the measuring chamber 18 and in which measuring chamber 18 a holding mechanism 3 is contained. In the holding mechanism the frame 4 for holding the flexible sheet-like material to be tested is provided. The holding mechanism 3 together with the frame 4 are identical to those depicted in Fig. 2, so that a special description thereof seems not to be necessary.

Holes 8 for introducing or discharging highly-purified air are provided in the measurement chamber 18. In the embodiment according to Fig. 3 the holes 8 for discharging highly-purified air, which is enriched with emitted particles, are provided in the centre region on the top surface 15 of the measurement chamber 18 and it is therefore not necessary to provide discharging holes 8 on each side of the flexible sheet-like material to be tested.

The speakers 2 are arranged on each side of the flexible sheet-like material to be tested and an exit for the acoustic waves is provided in each part 12 and 13 of the casing 1. This exit 10 is connected with an air tight tubing, which is not shown in Fig. 3.

During measurement the flexible sheet-like material to be tested is arranged in the centre between two particle emission free speaker membranes 9 according the embodiment of Fig. 3. By applying a periodic electric signal to the speakers, the air column between the speaker membranes transmits the vibrations to the sample and deforms it mechanically. If the two signals are shifted by π (180°) in phase, the transmitted acoustic power of both speakers will sum up at the position of the sample and increase the efficiency of the system. The acoustic waves exiting at the back of the speaker compartments 12, 13 are merged by means of an air tight tubing to insulate the whole system from the ambient laboratory air.

The air exiting through holes 8 from the measurement chamber 18 will be led to various aerosol characterizing and sampling instruments such as for example:
- a condensation particle counter 27 (Fig. 1) continuously determining the actual total particle concentration of particles in the measurement chamber,
- at sufficiently high concentrations, an electro-mobility spectrometer can be used for the determination of the particle number size distribution in the size range from a particle diameter of 2 nm up to 1,5 µm, with an additional spectrometer the said range can be extended to 300 µm,
- an electrostatic precipitator can be used to sample the emitted particles on an electron microscope carrier (standard 3,5 mm copper grid) for further structural analysis,
- the sampling of particles on a nuclepor filter for further chemical analysis is possible.

With such a system both the amplitude as well as the frequency of the mechanical stress will be continuously controllable. Therefore, the emission characteristics can be investigated over a broad spectrum of intensity and movement pattern of the mechanical stress applied to the sample.

As a matter of fact it is necessary that all electromechanical parts, i.e. sample changer/feeder, magnetic valves, wave form generator, power amplifier 32, aerosol measurement instrumentation 25, 26, 27, 30 etc. will be interfaced to and process controlled by a computer 29, but these electromechanical parts are known in the art.

Data analyses will be carried out automatically, as only a direct feedback mechanism ensures the proper operation of the whole system.

In this way a fully automated operation as well as a fully manual operation are made possible with the device according to the present invention thereby making the present concept feasible for scientific investigations and standard investigations, e.g. quality control as well.

In Fig. 4 a particle size distribution obtainable with a device according to Fig. 2 or 3 is shown. From this graph, the result of a particle size emission measurement with a device 1 according to Fig. 3 using a paper sheet as flexible sheet-like material can be gathered. It is clearly shown that particles between 110 and 500 to 700 nm are contained in a great amount on or in such a paper sheet.

## Claims

1. Process for quantitative determination of emissions, consisting of particulate matter, from surfaces of flexible sheet-like materials, wherein the flexible sheet-like material is fixed in a frame, introduced in a measurement chamber (18), flushed with highly-purified air and periodically moved by exposing to a mechanical stress, whereupon the particulate matter emitted by the flexible sheet-like material is fed by highly-purified air to a detection device (25, 26, 27, 29, 30), **characterized in that** the mechanical stress is generated by waves, especially pressure waves or sound waves.

2. Process according to claim 1, **characterized in that** the mechanical stress is exhibited on both sides of the frame (4) by at least one wave-generating device (2).

3. Process according to claim 1 or 2, **characterized in that** the waves of the wave-generating devices (3), which are arranged on opposite sides of the frame (4), are shifted by phase, especially shifted by 180°.

4. Process according to claim 1, 2 or 3, **characterized in that** the frequency and/or the amplitude of the waves are continuously controlled.

5. Process according to any of claims 1 to 4, **characterized in that** energy generated by the wave generating device(s) (2, 3) releases attached or absorbed particles, with a particle size of at least 2 nm, from the surface (6) of the flexible sheet-like material.

6. Process according to any of claims 1 to 5, **characterized in that** a continuous stream of highly-purified air is maintained during the exposure to mechanical stress and that after ending of the exposure to mechanical stress the measurement chamber (18) is further flushed with highly-purified air.

7. Process according to any of claims 1 to 6, **characterized in that** a stream of highly-purified air, which is enriched with positive and negative ions, for transporting the particulate matter emitted by the flexible sheet-like material, is continuously introduced into the measurement chamber (18).

8. Process according to claim 7, **characterized in that** the concentration of positive and negative ions in the stream of highly-purified air is at least 10⁵ ions/cm³, especially 10⁷ ions/cm3.

9. Process according to any of claims 1 to 8, **characterized in that** the flexible sheet-like material is selected from the group consisting of paper, cardboard, synthetic materials, foils, leather, cloth or fabric.

10. Device for quantitative determination of emissions, consisting of particulate matter, from surfaces of a flexible sheet-like material, comprising a casing (1), in which casing (1) a frame (4) for holding the flexible sheet-like material is introducible, at least one supply conduit for highly-purified air and at least one discharge conduit for a stream, consisting of highly-purified air and emitted particulate material, a mechanical stress generating device (2) and a detection and measurement unit (25, 26, 27, 29, 30), **characterized in that** the mechanical stress generating device (2) is selected from a pneumatic system or a sound waves producing system.

11. Device according to claim 10, **characterized in that** the casing (1) comprises at least two parts (12, 13), which are air tight connectable and that in at least one part (12, 13) one element of at least one mechanical stress generating device (2) is contained.

12. Device according to claim 10 or 11, **characterized in that** as mechanical stress generating device (2) at least two speakers are provided.

13. Device according to any of claims 10 to 12, **characterized in that** in one part (12, 13) of the casing (1) a frame holding device (3) is provided, in which frame holding device (3) the frame (4) is introducible or removable by a frame exchange mechanism.

14. Device according to any of claims 10 to 13, **characterized in that** the measurement unit contains different aerosol characterizing and sampling instruments, which are selected from a condensation particle counter (27), an electro-mobility spectrometer, a flow control unit (26), an electrostatic precipitator, an electron microscope and particle sampling elements, such as particle filters (30) or so on.
